# EUROPEAN PATENT SPECIFICATION

(11) **EP 2 856 166 B1**
(45) Date of publication and mention of the grant of the patent: **08.11.2017**
(21) Application number: 13797381.4
(22) Date of filing: 30.05.2013
(51) Int. Cl.: G01N 33/574, G01N 33/50, G01N 33/53, G01N 33/557, G06F 19/24, G06F 19/26, G06F 19/00

(54) **METHOD FOR THE DIAGNOSIS OF MAMMALS**
VERFAHREN ZUR DIAGNOSE VON SÄUGETIEREN
MÉTHODE POUR LE DIAGNOSTIC DE MAMMIFÈRES

(30) Priority: 01.06.2012 SE 1200341
(43) Date of publication of application: 08.04.2015
(73) Proprietor: Ridgeview Diagnostics AB, 751 83 Uppsala (SE)
(72) Inventor: ANDERSSON, Karl, 740 20 Vänge (SE); MALMQVIST, Magnus, 757 52 Uppsala (SE); LEBEL, Lena, 757 52 Uppsala (SE); BJÖRKELUND, Hanna, 753 24 Uppsala (SE); GEDDA, Lars, 752 41 Uppsala (SE)
(74) Representative: Brann AB
(86) International application number: PCT/SE2013/050627
(87) International publication number: WO 2013/180635

(56) References cited:
- WO-A1-2005/080967
- WO-A1-2010/033069
- US-B1- 6 297 018
- K. LEKPOR ET AL: "An evaluation of multidimensional fingerprinting in the context of clinical proteomics", PROTEOMICS - CLINICAL APPLICATIONS (WILEY-VCH VERLAG & CO, vol. 1, no. 5, 1 May 2007 (2007-05-01), pages 457-466, XP055231833, Weinheim FRG ISSN: 1862-8346, DOI: 10.1002/prca.200600890
- D. ALTSCHUH ET AL: "Deciphering complex protein interaction kinetics using Interaction Map", BIOCHEMICAL AND BIOPHYSICAL RESEARCH COMMUNICATIONS (ELSEVIER INC), vol. 428, no. 1, 1 November 2012 (2012-11-01), pages 74-79, XP055175490, New York NY USA ISSN: 0006-291X, DOI: 10.1016/j.bbrc.2012.10.008
- L. GEDDA ET AL: "Evaluation of Real-Time Immunohistochemistry and Interaction Map as an Alternative Objective Assessment of HER2 Expression in Human Breast Cancer Tissue", APPLIED IMMUNOHISTOCHEMISTRY MOLECULAR MORPHOLOGY (LIPPINCOTT WILLIAMS AND WILKINS INC), vol. 21, no. 6, 1 December 2013 (2013-12-01), pages 497-505, XP008175057, PHILADELPHIA, PA, USA ISSN: 1062-3345, DOI: 10.1097/PAI.0B013E318281162D
- ALTSCHUH D. ET AL.: 'Dechipering complex protein interaction kinetics using interaction map' BIOCHEM. BIOPHYS. RES. COMM. vol. 428, 2012, pages 74 - 79, XP055175490
- GEDDA L. ET AL.: 'Real-time immunohistochemistry analysis of embedded tissue' APPLIED RADIATION AND ISOTOPES vol. 68, 2010, pages 2372 - 2376, XP027265716

## Description

### FIELD OF INVENTION

The present invention relates to a method for the diagnosis or prognosis of mammals, i.e. humans or animals. More in particular, it relates to a method for the diagnosis of individuals or animals based on one or more tissue samples being subjected to probe which recognizes presence and quantity of predefined structures in said tissue sample.

### BACKGROUND OF THE INVENTION

Diagnostic procedures are crucial in many functions in the modern society. One of the most common is the diagnostic procedures performed at health care institutions (e.g. hospitals) with the purpose to determine if a patient has a particular disease, to monitor the progression of a disease, or to follow the effectiveness of treatment of a disease. For example, elevated concentration of the prostate specific antigen (PSA) in male blood is an indication of ongoing prostate cancer in the patient. However, this is not specific enough and there is need for better methods to analyze different modifications of PSA to improve the clinical decision based on analytical results.

Other diagnostic procedures include, but are not limited to, the diagnosis of cancer based on ocular analysis of stained tissue biopsies, diagnostics of cattle prior to slaughter in order to produce safe food, diagnostic procedures in veterinary sciences with the purpose of treating sick animals, medical imaging of tumors in animal or humans and the like.

Whenever the sample structure is complex, most of the currently used methods must use reagents that are highly specific to amplify the signal from one component in the complex sample. Such complex structures can be cells in a tissue sample.

One particular method for diagnosis is immunohistochemistry (IHC). Diagnostic IHC procedures are developed for a multitude of diseases, most notably for cancers.

In brief, IHC is a method wherein a thin slice of tissue is placed onto a microscope glass slide followed by staining of selected receptors. An image is made of the stained tissue slice and a trained operator is judging if the tissue contains staining patterns indicative of disease. Even though IHC is used world-wide and has improved the possibility to diagnose serious diseases like cancer, general IHC methodology still suffers from poor repeatability and long tissue preparation protocols (as evident in the report "Current issues in ER and HER2 testing by IHC in breast cancer." by Allen M Gown published in Modern Pathology 2008 May;21 Suppl 2:S8-S15).

Immunohistochemistry is one of the dominant methods for analysis of tissue slices. Being used in the majority of major hospitals, it is a well known method for persons skilled in the art of tissue analysis. In brief, IHC is a method for localizing proteins in cells of a tissue section by use of antibodies binding specifically to antigens in biological tissues. IHC staining is commonly used in the diagnosis of abnormal tissue such as tumors. Specific molecular structures on or in the cells in the tissue are characteristic of particular cellular events indicative of disease. In order to visualize the antibody-antigen interaction, the antibody can be tagged to a detectable label, for example a fluorophore such as FITC, rhodamine, Texas Red or any other fluorescent moiety. In the procedure thin (typically 20 µm) slices are taken of the tissue of interest. The tissue typically originates from surgery or biopsies, and is commonly paraffin embedded. The tissue is then treated to rupture the membranes, usually by using a detergent (e.g. Triton X-100). After these steps, the tissue slice is prepared for antibody treatment, which typically follows an indirect approach. The indirect approach involves a primary (unlabeled) antibody which reacts with tissue antigen, and a secondary (labeled) antibody which reacts with the primary antibody. The secondary antibody is normally labeled with a fluorescent moiety or an enzyme. IHC is a powerful detection technique and is capable of showing exactly where a given protein is located in the tissue sample.

IHC is widely used in many fields of biology, e.g. in the neurosciences, enabling researchers to examine protein expression within specific brain structures and in diagnostic surgical pathology for typing tumors (e.g. carcinoma vs melanoma). The result of an IHC analysis is an image of the tissue slice with areas containing certain targeted receptors stained in a distinguishable color. As such, IHC is an end-point measurement, i.e. it is only possible to detect the status of the antibody-antigen interaction at one point in time. IHC also suffers from the often manual interpretation of images; trained operators may disagree on the extent and intensity of the staining of the very same tissue slide, leading to uncertainties when comparing results across operators and laboratories. Another major disadvantage of current IHC protocols is that it is impossible to show in IHC that the staining corresponds with the protein of interest. A description of IHC used in clinical practice is available in the report "Diagnostic evaluation of HER-2 as a molecular target: an assessment of accuracy and reproducibility of laboratory testing in large, prospective, randomized clinical trials." By Press MF, Sauter G, Bernstein L, Villalobos IE, Mirlacher M, Zhou JY, Wardeh R, Li YT, Guzman R, Ma Y, Sullivan-Halley J, Santiago A, Park JM, Riva A, Slamon DJ. Published in Clinical Cancer Research 2005 Sep 15; 11(18):6598-607.

One common method for using IHC in clinical practice is to classify the staining into different levels, typically 0 for negative, + for detectable, ++ for clear staining and +++ for strong staining. This semi-quantitative scale may be used for selecting treatment. In the case of breast cancer, about one in four tumors have the oncogene 15 product HER2 overexpressed, and in such cases it may be advisable to prescribe the therapeutic antibody Herceptin®. Currently, patients for which the tissue sample score +++ for HER2 expression are usually prescribed Herceptin. Patients for which the tissue sample score ++ are typically re-evaluated using a different technology (fluorescent in-situ hybridization; FISH). If the tissue is positive also in FISH, Herceptin is usually prescribed. Patients for which the tissue sample score ++/FISH- , +, or - are usually not prescribed Herceptin.

Recent developments of IHC includes the ability to follow the binding process of an antibody to the tissue specimen in real-time, as described in the report "Real-time immunohistochemistry analysis of embedded tissue" by Gedda L, Björkelund H, and Andersson K, published in Applied Radiation and Isotopes 68 (2010) 2372-2376.

The method Interaction Map was further technically evaluated as described in the publication "Deciphering complex protein interaction kinetics using Interaction Map" as published by Altschuh et al in Biochem. Biophys Res Comm vol 428, 2012, pp74-79.

Some of the details in the present applications were disclosed and further elaborated, after the priority date of the present invention, in the publication "Evaluation of real-time immunohistochemistry as an alternative objective assessment of HER2 expression in human breast cancer tissue" as published by Gedda an co-authors in Applied immunohistochemistry molecular morphology vol 21 no 6 1 December 2013 pp 497-505.

A method for applying fingerprints in a general sense for the determination of status of an individual through applying a mass-spectroscopy method on a blood sample was disclosed by Lekpor and co-authors in "An evaluation of multidimensional fingerprinting in the context of clinical proteomics", as published in Proteomics Clin. Appl. 2007, 1 (5), 457-466. disclosure discuss the general term fingerprint from a mapping of individual proteins and relative protein concentrations in blood as assessed using concentration determination through mass spectrometry. The disclosure is similar to the present invention in that a large number of data points is collected and handled as a fingerprint or pattern which comprises information suitable for determining the status of an individual, but differs from the present invention in that it is based on a functionally insensitive method, meaning that a misfolded protein would appear equal to a properly folded protein in the method of Lekpor, but where the present invention as based on a functional methodology would readily detect misfolded proteins as absence of function in a predetermined region of the Interaction Map fingerprint. Misfolded proteins are common in a variety of diseases, such as Alzheimers disease.

Recent development of analysis of real-time molecular interaction data includes the method "Interaction Map", as described in the patent application US2011195434 "METHOD FOR THE ANALYSIS OF SOLID OBJECTS ".

The development of a diagnostic method is generally complicated, irrespective of which underlying detection technology used or which type of patient material is used. Not only must the detection technology be adapted to the patient sample to be characterized as indicating disease or not, it must be rigorously validated to provide sufficiently accurate results to be of clinical use.

### SUMMARY OF THE INVENTION

The object of the present invention is to provide a reliable method of assessing or determining if a subject mammal (human or animal) suffers from a specified condition. This object is met by the method defined in claim 1. The method according to the invention is particularly useful for but not limited to the diagnosis of cancer by use of antibody based probes.

The method is furthermore usable in the development of methods for diagnosis based on analysis of biological samples, which is facilitated by the invention. Thereby, the analysis employed comprises use of one or more probes that interacts with structures on said biological sample. An embodiment the invention comprises the method as described above wherein said oncogene product is a growth factor receptor.

In another embodiment the invention comprises the method as

described above wherein the probe is an antibody.

### BRIEF DESCRIPTION OF THE DRAWINGS

The present invention will be disclosed in closer detail in the description and example below, with reference to the accompanying drawing, in which
Figure 1 shows a schematic representation of the method of the invention;
Figure 2 shows a suitable instrument, known in prior art, for performing the measurement in the quality control method;
Figure 3 illustrates a solid support with multiple tissue samples attached;
Figure 4 illustrates three binding curves as simultaneously measured on a solid support holding three different tissue samples;
Figure 5 illustrates Interaction Maps from one positive reference tissue sample measured in eight different solid supports;
Figure 6 illustrates Interaction Maps from tissue samples from eight patients; and
Figure 7 illustrates a suitable region for extracting data for classification in Example 1.
Figure 8 illustrates (a) one binding curve measured using an antibody sourced from an alternative vendor and (b) the corresponding Interaction Map;

### DETAILED DESCRIPTION OF THE INVENTION

For the purpose of the present application, and for clarity, the following definitions are made:

A "biological sample" is defined as a small portion of tissue excised from an individual or an animal, including but not limited to portions of body fluid (e.g. blood sample, saliva sample, spinal fluid sample and similar), and solid tissue samples (e.g. biopsies, excess material from surgery, skin grafts and similar).

"Tissue sample" is defined as a biological sample comprising a solid biological object and include, but is not limited to, excess material from surgery, biopsies, embedded tissue samples and sections thereof. A tissue sample is thinner than 1 mm, and is typically thinner than 0.1 mm. The tissue sample further has an area less than 100 cm², and typically said area is greater than 1 mm² and less than 10 cm². The tissue sample under analysis is attached to a solid support and the predefined probes designed to interact with structures on the tissue sample are present in a liquid that is in contact with said solid support. The tissue sample under investigation can be prepared using different methods. One common method is to embed the tissue sample in paraffin according to common IHC protocols, followed by slicing thin sections for attachment to the solid support and analysis using the method of this invention. It is further possible to use fresh-frozen tissue, sliced into thin sections, attached onto the solid support and analyzed using the method of this invention. Tissue sample can also be blood samples analyzed either as blood smears fixed in different ways or as living cells by flow cytometry.

A "probe" is defined as having at least two characteristic properties: First, a probe has to attach to or interact with a structure which is searched for in the tissue sample under investigation. Second, it must be possible to detect, in a time resolved fashion, the probe interacting with said structure on said tissue sample. A typical probe for a biological object may be a protein known to specifically interact with a certain receptor, the presence of said receptors in the tissue sample being known in advance to be indicative of disease. Said probe may further have a fluorescent tag (e.g. FITC, Cy2, Cy3, Cy5, Texas Red, or any other fluorescent tag) attached for simple detection of the amount of probe bound to said tissue sample. Possible probes for use in analytical or diagnostic procedures include but are not limited to, macromolecules (e.g. proteins, DNA, RNA), antibodies, aptamers, affibody molecules, nanobodies, peptides and other chemical compounds and any species that can be dissolved in liquid. The probe may have some sort of label attached. Suitable labels include, but are not limited to, radioactive labels and fluorescent labels.

A "disease-related target" is defined as a structure on said biological sample, said structure being characteristic for a particular disease. Disease-related targets include, but are not limited to, surface structure changes related to neurological disease as exemplified by Gladys E. Maestrea, Barbara A. Tatea, Ronald E. Majochaa, and Charles A. Marotta, in the report "Membrane surface ruffling in cells that over-express Alzheimer amyloid β/A4 C-terminal peptide" published in Brain Research (621(1):145-149, 1993, which is incorporated by reference herein. Disease-related targets further include, but are not limited to, surface structure changes related to cancer, as illustrated by J Carlsson in the report "Potential for clinical radionuclide-based imaging and therapy of common cancers expressing EGFR-family receptors" published in Tumour Biol. 2012, which is incorporated by reference herein. Disease-related targets further include, but are not limited to, surface structure changes related to diabetes as exemplified by Green-Mitchell SM, Cazares LH, Semmes OJ, Nadler JL, Nyalwidhe JO in the report "On-tissue identification of insulin: in situ reduction coupled with mass spectrometry imaging" published in Proteomics Clin Appl. 2011 Aug;5(7-8):448-53, which is incorporated by reference herein.

An "oncogene product" is defined as a disease-related target for cancers, i.e. a molecule abundantly present in a cancer cell, and typically present in smaller quantities in normal cells. An oncogene product as defined in this invention can be the translated product of an over-expressed gene in a cancer cell. Another possibility is a molecule that is abundant in both normal and cancer cells, but that in cancer cells have different post-translational modifications (including, but not limited to different glycosylation pattern, ubiquitination pattern, and/or methylation pattern). Yet another possibility includes mutations of a molecule common in normal cells. Still another possibility is a molecule present in normal cells that in cancer cells change conformation in different ways after the processes of preparation of thin sections due to unfavorable or changed protein properties or environment. Yet another possibility includes proteins that are modified in primary, secondary or tertiary protein structure in cancer cells. Due to the complex nature of cancer diseases, it is understood that an oncogene product is commonly valid only for a particular type of cancer. Thus, an oncogene product indicative of disease in one type of cancer may not be useful for other types of cancer. The oncogene product is commonly a receptor, overexpressed due to genetic instability of the cancer disease but can also be an intracellular component in signal-transduction chains or a protein such as p53 that play a fundamental role in cancer cell regulation and is frequently mutated in different cancer forms. Oncogene products include, but are not limited to, estrogen receptor (ER) and its targets; human epidermal growth factor receptor 2 (HER2/ErbB2), epidermal growth factor receptor (EGFR/HER1); progesterone receptor (PR), cytokeratin 5, cytokeratin 6, cytokeratin 14, cytokeratin 17), BRCA1, BRCA2, p53, alpha-B-crystallin, mutationally inactivated Fbxw7, elevated expression of E2F3 and Cyclin E genes, cyclin E, N-cadherin, vimentin, FOXC2, Twist, Slug, Snail, LBX1, Src family tyrosine kinase LYN, tyrosine kinases VEGF receptor (VEGFR)1/Flt1 and VEGFR2/KDR/Flk1 including Neuropilin coreceptors, amplified or overexpressed genes FGFR2, BUB3, RAB20, NOTCH3, and PKN1, all recognized as related to cancer as discussed in "Minireview: Basal-Like Breast Cancer: From Molecular Profiles to Targeted Therapies" by Daniel J. Toft and Vincent L. Cryns published in Mol Endocrinol, February 2011, 25(2):199-211 (which is incorporated by reference herein).

Oncogene products further include, but are not limited to, the four members of the ErbB family: epidermal growth factor (EGF) receptor (also termed ErbB1/HER1), ErbB2/Neu/HER2, ErbB3/HER3 and ErbB4/HER4, all recognized as related to cancer as discussed in "The ErbB signaling network: receptor heterodimerization in development and cancer" by MA Olayioye, RM Neve, HA Lane, and NE. Hynes published in EMBO J. 2000 July 3; 19(13): 3159-3167 (which is incorporated by reference herein).

Oncogene products further include, but are not limited to, growth factor receptors (including PDGF, IGF, FGF, VEGF, and EGF receptors), mutant Ras oncogenes, mutant (dysfunctional) TGF-beta receptors, c-Myc oncoprotein, IGF-1R, IL-3R, FAS receptor, TNF-R1, Bcl-2, Bcl-XL, Bcl-W, E-cadherin, all recognized as related to cancer as discussed in "The Hallmarks of Cancer" by D Hanahan and RA Weinberg published in Cell, Vol. 100, 57-70, January 7, 2000 (which is incorporated by reference herein).

Oncogene products further include, but are not limited to, Calumenin precursor, Procollagen-lysine,2-oxoglutarate 5-dioxygenase 1 precursor, Calcium-binding mitochondrial carrier protein aralar, NADPH-cytochrome P450 reductase, Galectin-1, Fibulin-1 precursor, Peripherin, Transferrin receptor protein, Band 4.1-like protein, Complement clq subcomponent, b chain precursor, Eukaryotic translation initiation factor 4 γ 2; eIF-4G, Dolichyl-diphosphooligosaccharide-protein glycosyltransferase subunit STT3A, Fascin; 55-kDa actin-bundling protein, Hexokinase-2, S100 calcium-binding protein A9, ERO1-like protein α precursor, S100 calcium-binding protein A8, Coagulation factor xiii a chain precursor, Ig κ chain v-iv region precursor, Superoxide dismutase [mn], mitochondrial precursor, Ig γ-4 chain c region, Kynureninase, α Crystallin B chain, Solute carrier family 2, facilitated glucose transporter member, Putative transmembrane protein nmb precursor, Ig γ-3 chain c region, Caspase-14 precursor, Cathepsin B precursor, Fatty acid-binding protein, brain, IgGFc-binding protein, H liver carboxylesterase 1 precursor, all recognized as related to cancer as discussed in "Differential Protein Expression Profiles in Estrogen Receptor-Positive and -Negative Breast Cancer Tissues Using Label-Free Quantitative Proteomics" by K Rezaul, JK Thumar, DH Lundgren, JK Eng, KP Claffey, L Wilson and DK Han published in Genes Cancer. 2010 March; 1(3): 251-271 (which is incorporated by reference herein).

Oncogene products further include, but are not limited to, Adrenocorticotropin (ACTH), Akt-pS473, Alpha-1-Antitrypsin, Alpha-1-Fetoprotein, AMACR, Amyloid A, Androgen Receptor, Bax, B-Cell-Specific Activator Protein, BCL2 Oncoprotein, BCL6 Protein, BCL10 Protein, Beta-Amyloid, Beta-Catenin, BRCA1, Bromodeoxyuridine, C1q Complement, C3c Complement, C4c Complement, C5b-9 (TCC), CA 19-9, CA 125, Calcitonin, Caldesmon, Calponin, Calretinin, Carcinoembryonic Antigen (CEA), Carcinoembryonic Antigen (CEA), CD1a, CD2, CD3, CD3, CD4, CD5, CD7, CD8, CD10, CD 14, CD 15, CD19, CD20cy, CD21, CD23, CD30, CD31, Endothelial Cell, CD34 Class II, CD35, CD43, CD44, Phagocytic Glycoprotein-1, CD45, Leucocyte Common Antigen, CD45R0, CD45RA, CD56, CD57, CD61, Platelet Glycoprotein IIIa, CD68, CD68, CD68, CD79α, CD79αcy, CD99, MIC2 Gene Products, Ewing's Sarcoma Marker, CD105, Endoglin, CD117, c-kit, CD138, CD235a, Glycophorin A, CD246, ALK Protein, CDX2, c-erbB-2 Oncoprotein, Chorionic Gonadotropin (hCG), Chromogranin A Collagen IV, COX-2, Cyclin D1, Cytokeratin, Cytokeratin 5/6, Cytokeratin 7, Cytokeratin 10, Cytokeratin 10/13, Cytokeratin 17, Cytokeratin 18, Cytokeratin 19, Cytokeratin 20, Cytokeratin, D2-40, Desmin, E-Cadherin, EGFR Wild-Type, EGFR-pY1197

Phosphorylation Site Specific, Epithelial Antigen, Epithelial Membrane Antigen (EMA), Epithelial-Related Antigen, Estrogen Receptor α, Estrogen Receptor α, Estrogen Receptor β1, Fascin, Fibrinogen, Follicle-Stimulating Hormone (FSH), Follicular Dendritic Cell, Gastrin, Glial Fibrillary Acidic Protein (GFAP), Glucagon, Glycophorin C, Granzyme B, Gross Cystic Disease Fluid Protein-15, Growth Hormone (hGH), Hepatocyte, HER2-pY 1248, Phosphorylation Site Specific, HER3, HLA-ABC Antigen, HLA-DP, DQ, DR Antigen, HLA-DR Antigen, Alpha-Chain, Human Immunodeficiency Virus (HIV) p24, IMP3, Inhibin α, Insulin, Ki-67 Antigen, Ki-67 Antigen, Ki-67 Antigen, Lambda Light Chains, Laminin, Laminin-5 Gamma-2 Chain, LAT Protein, Leukaemia Hairy Cell, Luteinizing Hormone (LH), Lysozyme EC 3.2.1.17 (Muramidase), Macrophage, MAGE-C1, Mammaglobin, Mast Cell Tryptase, Matrix Metalloproteinase 9 (MMP-9), MCM3 Protein, Melan-A, Melanosome, Mesothelial Cell, Metallothionein, MITF, MUM1 Protein, MutL Protein Homolog 1, Myelin Basic Protein, Myeloid/Histiocyte Antigen, Myeloperoxidase, MyoD1, Myogenin, N-Cadherin, Neurofilament Protein, Neuron-Specific Enolase (NSE), Neutrophil Elastase, Nucleophosmin, p21WAF1/Cip1, p27Kip1, p53 Protein, Papillomavirus (HPV), Parvovirus B19, PGP 9.5 , Placental Alkaline Phosphatase, Progesterone Receptor, Prolactin, Proliferating Cell Nuclear Antigen, Prostate-Specific Antigen (PSA), Prostate-Specific Membrane Antigen (PSMA), Prostatic Acid Phosphatase, Prostein, PTEN, Renal Cell Carcinoma Marker, Ribosomal Protein S6-pS240, Phosphorylation Site Specific, S100, S100A4, Serotonin, Somatostatin, Survivin, Synaptophysin, Tau, Terminal Deoxynucleotidyl Transferase (TdT), Thrombomodulin, Thymidylate Synthase, Thyroglobulin, Thyroid Peroxidase (TPO), Thyroid-Stimulating Hormone (TSH), Thyroid Transcription Factor (TTF-1), Tissue Inhibitor of Metalloproteinases 1, Topoisomerase IIα, Tyrosinase, Ubiquitin uPAR, Vascular Endothelial Growth Factor (VEGF), Villin, Vimentin, Vimentin, Von Willebrand Factor, Wilms' Tumor 1 (WT1) Protein, ZAP-70, each of these oncogene products having a commercial antibody probe available from Dako Denmark A/S, Glostrup, Denmark.

The term "condition" as used in the context of the present invention of classifying individuals or animals as having a particular condition or not denotes a status which commonly is related to disease. One example of a possible condition is that the individual or animal may have cancer. Possible conditions include, but are not limited to, cancer, diabetes, viral infection, bacterial infection, allergy, sepsis, and rheumatoid arthritis. A condition need not be a disease, and hence also include (but is not limited to) the determination of if a female individual or animal is pregnant and the assessment of prognosis of an individual or animal which is already known to have a condition such as cancer.

The term "expression level" denotes the number of disease-related target molecules being present in or on the biological sample. The expression level is typically presented as "number of disease-related target molecules per cell".

The term "characteristic value" refers to a value derived from one or more features of a multidimensional representation or multidimensional fingerprint of measured data for one probe. One example of a characteristic value is the area under the measured curve of the compound interacting with a biological structure. Another example of a characteristic value is the peak position of the dominant peak in an interaction map calculated for a compound interacting with a disease related target. The characteristic value is typically a scalar numerical value.

The term "primitive curves" denotes a plurality of curves, which in linear combination may reproduce a majority of the expected possible measured curves. For example, in the case of molecular interactions a suitable set of primitive curves includes (but is not limited to) a collection of curves where each curve represents a monovalent interaction with unique pair of association rate and dissociation rate values. Given a sufficient number of such primitive curves, it is possible to reproduce the data obtained from the measurement of a molecular interaction with a linear combination of said primitive curves. The concept of primitive curves is equivalent to the concept of a base vector system, wherein any monotonous curve can be expressed as a linear combination of a base vectors, provided that the base vector system is complete.

The term "multidimensional fingerprint" refers to a non-scalar numerical representation of one or more time-resolved measurements. A multidimensional fingerprint may be constructed through the use of primitive curves, wherein the time-resolved measurement is reconstructed as a linear combination of said primitive curves. The coefficients in the linear combination, sometimes referred to as weights, can be utilized as the multidimensional fingerprint. A multidimensional fingerprint comprises preferably more than 10 different numerical values, even more preferably more than 100 different numerical values.

Within an Interaction Map, there is sometimes a feature present denoted "peak". A "peak" refers to a region in the Interaction Map with high density, which when plotted like a topographic interaction map looks like a peak or a hill. A peak has a position or center which represent the average binding parameters (log10(ka) and log10(kd)). A peak also has a width which reflects the heterogeneity, where a small width (i.e. a narrow peak) indicates a homogenous interaction. A peak further has a weight which is a measure of the density or contribution of the peak to the total interaction. Interaction Maps typically produce fingerprints in normalized units, meaning that the sum of the weight for the complete map is 100%, which in turn means that a peak with e.g. 80% weight contributes to the majority of the detected interaction.

Generally, the invention in its first aspect is based on the provision of six characteristic components. These six components are:
- Obtaining a of biological sample from a human or an animal believed to be have a condition;
- Providing one or more probes that are known to interact with a disease-related target known to be associated with said condition, said probe possibly being labeled with a detectable marker;
- Conducting a time resolved measurement of the interaction between the probes and the disease-related target on said biological sample;
- Calculating a representation of the time resolved measurement, where said representation provides a multidimensional fingerprint of the nature of the interaction of the probe with the disease-related target on said biological sample;
- Extracting a pre-defined region or pre-defined feature of said multidimensional fingerprint;
- Applying a classification algorithm which uses information within said region or said feature of said fingerprint to assess the status of a biological sample, said classification algorithm being capable of assessing if an individual or an animal has a selected condition or not.

These six components are usually defined in the much larger process of developing the diagnostic method as such, which includes at least the following nine steps:
- Selecting a diagnosis for which to develop a diagnostic method,
- Obtaining a set of biological samples from a cohort of individuals or animals, known in advance to have the selected condition or diagnose and believed to carry a disease-related target (the case group),
- Obtaining a set of biological samples from a cohort of individuals or animals known to be devoid of the selected condition or diagnosis and believed not to carry a disease-related target (the control group),
- Providing one or more probes that are known to interact with said disease-related target, said probe possibly being labeled with a detectable marker,
- Providing a time resolved measurement of the interaction between the probes and the disease-related target on said biological sample,
- Providing a method for representing the time resolved measurement, where said representation provides a multidimensional fingerprint of the nature of the interaction of the probe with the disease-related target on said individual or animal.
- Providing a defined region or a defined feature of said multidimensional fingerprint
- Identifying a classification algorithm which uses information within said region of said fingerprint to asses a statement on the status of a tissue sample.
- Finally, applying the process of measurement on biological samples, analysis by use of multidimensional fingerprint, and use of said classification algorithm on new individuals or animals in order to assess if said new individuals or animals are likely to have said diagnosis.

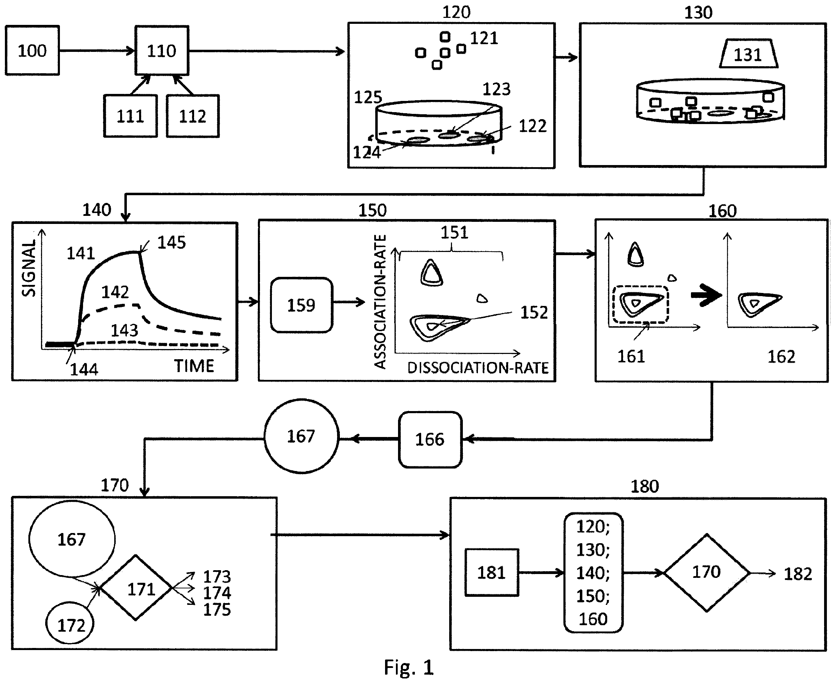

The method used for analytical or diagnostic or prognostic purposes can, as exemplified in the context of tissue sample diagnosis of cancer, be described as a nine-step procedure which is outlined in Figure 1. In the first step, a condition or diagnosis (100) is selected for which a diagnostic method is to be developed. In the second step, a plurality of tissue samples (110), some known to represent the selected diagnose (111; sometimes known as cases) are obtained, and some known not to represent the selected diagnose (112; sometimes known as controls), are collected.

In a third step (120), one or more tissue samples (122-124) are attached in a non-overlapping manner to a solid support (125) and a suitable probe (121) known to interact with a disease related target (which in turn is known to be associated with the selected condition or diagnose) dissolved in liquid is made available. Preferably, at least one of said tissue samples is a reference tissue known to express the desired disease related target (in this example an oncogene product) and another one of the tissue samples is a reference tissue known not to express the desired disease related target (in this example an oncogene product).

In the fourth step (130), the liquid containing probe (121) is put in contact with the solid support (125) and the tissue samples (122-124). When the liquid is in contact with the solid support (125), a measurement device (131) is activated in order to measure in a time resolved fashion the amount of probe (121) that has bound to the oncogene product in the tissue samples (122-124). After a predetermined time, the liquid containing probe (121) may be replaced with liquid without probe.

In the fifth step (140), the readings from the measurement device is presented as a binding curves, one for each tissue sample (141-143) which shows both the presence of interaction between probe and oncogene product and the rate of formation of probe - oncogene product complexes as a curve representing amount of probe bound to the tissue sample over time. The time points for probe addition (144) and probe removal (145) are identified in the binding curves unless they are known in advance.

In the sixth step (150), each measured binding curve (141-143) is processed in a processor (159), said step of processing comprising performing a computation to bring about a conversion of said binding curve into an interaction map analysis (as defined in the patent application US2011195434). An interaction map can typically be displayed as a topographic map, where the surface of triplets (typically [association rate value, dissociation rate value, weight value]) is plotted as a contour plot (151) or a greyscale plot where a dark area indicates elevated weight for the corresponding [association rate value, dissociation rate value]. The map can also be described as a collection of "hills" or "peaks" where each peak (152) in this plot means that the corresponding association and dissociation rate values have elevated weights, which means that the binding curve subjected to analysis (any of 141-143) is partly composed of an interaction of the corresponding association and dissociation rate values, which in turn means that the probe (121) is interacting with the oncogene product of the tissue sample (122) with the corresponding association and dissociation rate values.

In the seventh step (160), one or more predetermined region(s) (161) in the interaction map are extracted for classification, thereby excluding any probe - oncogene interactions known in advance not to be of use for classification of said tissue sample. Alternatively, the interaction map is processed in a processor (166), said step of processing comprising performing a computation for extraction of one or more predefined features from a predetermined region of said interaction map so as to generate a characteristic value (167) based on said features.. Possible predefined features includes, but is not limited to, the total number of peaks in the interaction map, the weight of the biggest peak in relationship to all other peaks, and the relative heights of two defined peaks.

The reduced interaction map (162), or the extracted feature(s), or the characteristic value(s) (167), is then used in the eighth step (170) in a classification algorithm (171) the tissue sample, possibly together with further data (172). The classification algorithm may be a simple comparison of if a single value is greater than a predetermined threshold, such as if the biggest peak alone contributes to more than 70% ot the total density of the interaction map, to mention one example. The classification algorithm may also be a neural network, a linear discriminant classifier, a support vector machine, a k-nearest neighborhood classifier, or any other algorithm capable of classifying a set of input data into at least two different classes based on features in said input data. The output of the classification algorithm (171) is exemplified as three different classes (173, 174, 175), which for example could represent the classifications [healthy; uncertain; disease] of a tissue sample. The additional data (172) that the classification algorithm may use can be derived from the comparison of the different binding curves in the measurement, in particular if one or more reference samples are included. If, for example, binding curve 141 represents a positive reference sample, binding curve 142 a tissue sample with unknown properties and binding curve 143 a negative reference sample, the relative magnitudes of the three binding curves could be used as input for the classification algorithm. The unknown tissue sample 142 would, in this hypothetical example, by comparing signal levels of binding curves be classified as approximately 50% of positive control, and the accompanying interaction map would indicate if the signal measured for the tissue sample has the same interaction origin as for the positive reference sample. In some diagnostic procedures, the difference between a favorable status of a tissue sample (e.g. acceptable quality or benign tumor) is constituted by the relative fraction of related oncogene products in the tissue sample. In oncology, over-expression, mutations or modifications of certain receptors is known indications of disease, which in fact is an alteration of the relative abundances of the receptor plethora of a cell.

In the ninth step (180), the method for diagnosis as developed in the first eight steps is applied onto one or more patients with unknown status (181) by conducting steps three to eight (120, 130, 140, 150, 160 ,170) to produce an assessment (182) about the patient status regarding the selected diagnosis.

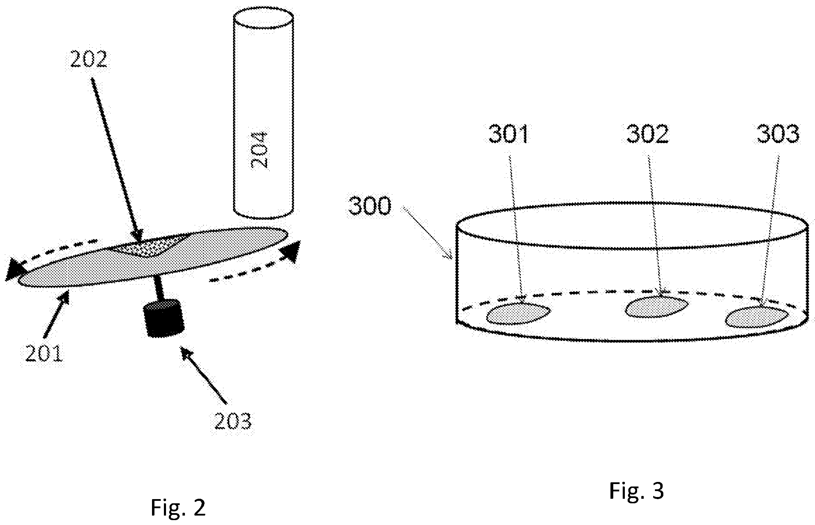

There are several methods available for the measurement of the rate of complex formation and the magnitude of formed complexes between a probe and a tissue sample. Methods based on Quarts Crystal Microbalance and similar techniques are available, as described in the report "A survey of the 2006-2009 quartz crystal microbalance biosensor literature." by Becker B and Cooper MA, published in J Mol Recognit. 2011 Aug;24(5):754-87 (which is incorporated by reference herein). Time resolved confocal microscopy and similar imaging techniques are alternative methods, as described by Praus P, Kocisová E, Mojzes P, Stepánek J, Seksek O, Sureau F and Turpin PY in the report " Time-resolved microspectrofluorometry and fluorescence imaging techniques: study of porphyrin-mediated cellular uptake of oligonucleotides." Published in Annals of the New York Academy of Sciences 2008;1130:117-21 (which is incorporated by reference herein). Flow cytometers (for example FACSCalibur as manufactured by Beckton Dickinson, Franklin Lakes, NJ, USA) are suitable for use on some types of biological samples, such as blood samples. A preferred method for completing step 130 in figure 1 has been previously disclosed [WO2005080967, which is incorporated by reference herein] and is schematically described in figure 2. In brief, the method relies on a solid object (202) being immobilized to a defined area on a solid support (201), denoted an "active area". On the same solid support, there is also a reference area (in this case opposite to the active area). A liquid containing a dissolved probe is in contact with the solid support to enable an interaction between object and probe. Furthermore, the solid support is inclined and slowly rotated using a motor (203). Over the elevated portion of the solid support, a detector capable of detecting the label attached to the probe used is mounted (204). Said detector is typically not in contact with the solid support, but registers e.g. emitted radiation of radioactive nuclides or emitted light from fluorescent labels. When the active area passes the detector, an elevated signal will be registered in case the ligand has bound to the target. For each rotation, a binding level value can be calculated by subtracting the detected signal from the reference area from the detected signal from the active area. When collecting binding level values from a series of rotations, a time resolved binding curve is obtained. In a general sense, the preferred device for completing step 130 in figure 1 as disclosed in WO2005080967 comprises a solid support to which the tissue samples are attached in a non-overlapping manner, a probe dissolved in liquid being in contact with the solid support, a detector capable of detecting an interaction between the probe and the oncogene product on the tissue sample. This device is arranged to temporarily reduce the amount of liquid on the part of the solid support which is in the field of view for the detector. The solid support should further have at least one defined area which is devoid of tissue sample or alternatively has a tissue sample known to be devoid of the oncogene product. This can be achieved by, for example, rotating a circular petri dish at inclined angle as illustrated in Figure 2. Another non-limiting possibility is to tilt an essentially flat solid support periodically back and forth, and activate the detector only for the elevated portion of the solid support.

The processing of data obtained from time-resolved measurements is carried out in a processor which performs a computation to create or calculate a multidimensional fingerprint or more particularly an interaction map. Said processing is typically performed using a non-transitory computer readable medium comprising instructions for causing a computer to perform steps required to calculate said multidimensional fingerprint or interaction map. In a similar manner, the processing of a multidimensional fingerprint or interaction map in a processor is typically performed using a non-transitory computer readable medium comprising instructions for causing a computer to perform steps required to calculate features and also the characteristic value.

In the case of tissue sample analysis, the probe may interact with several similar oncogene products. For example, cancerous cells in the tissue may express a certain receptor in abundant amounts and with mutations in the amino acid sequence of the receptor. The interaction map analysis is capable of deconvoluting several parallel probe-oncogene interactions and opens up for use of the heterogeneous nature as a decisive element in the classification algorithm of the current invention. For example, when using an antibody labeled with a fluorescent moiety as probe, said antibody being known to interact with a common oncogene product (for example the HER2), it is possible that the antibody interacts with the oncogene product in cancer cells in one way, and interacts with similar structures on normal cells in another way. Interaction map analysis would potentially deconvolute the interaction to cancer cells and normal cells into two separate peaks or hills, making it possible to use presence or magnitude of the peak related to cancer cells alone as input to the classification algorithm, thereby disregarding the probe interaction with the normal cells. Hence, the method of Interaction Map makes it possible to separate the signals from the different sources. In current methods like IHC, such a deconvolution is impossible because only the fact that the probe interacts with the oncogene is utilized for classification, not how the probe interacts with the oncogene.

The use of an in situ positive control, i.e. a positive control being simultaneously subjected to the same liquid containing a suitable probe as the biological sample, makes it possible to extend the information content. It is possible to use the positive control as a verification of that the assay works, i.e. that the correct liquid containing the correct probe has been added to the biological samples, and rejecting the measurement in cases where the positive control results do not meet predefined levels. It is also possible to use the positive control sample for the definition of a region in a multidimensional fingerprint wherein to analyze the biological sample(s). As one non-limiting example, in cases where the multidimensional fingerprint is Interaction Map and if the probe binds to the disease-related target in a manner that produces one dominant peak, the positive control could be used to define the positive feature to search for in the adjacent biological sample(s). The positive control could also be used for defining a region in the multidimensional fingerprint wherein the positive control feature is located in a particular measurement. Hence, the calculation of a characteristic value could include using a region in the interaction map of the positive control defined by for example the dominant peak, extracting the corresponding data from the interaction map for the biological sample. Optionally, it is even possible to compare features within the regions extracted from the positive control and the biological sample interaction maps, so as to use a relative value as characteristic value (e.g. the strongest intensity or weight found in the two regions presented as a quotient, to mention one non-limiting example). This would produce a robust assessment, because pipetting errors when producing the liquid containing the probe will result in slightly different concentrations which in turn may shift the location of the feature in the multidimensional fingerprint, and by quantifying in situ the location and appearance of the positive feature a method more robust to unexpected error is achieved. The same reasoning applies to the use of a negative control, wherein the multidimensional fingerprint of the negative control data could be used to define a feature for the negative case, and by comparing the multidimensional fingerprints for the biological samples with the negative control, a biological sample which is "not negative" could be defined as positive.

In current methods like IHC an attempt to stain a receptor may result in many types of staining appearance, including (but not limited to) "specific staining", "membrane staining", "periplasmic staining", "cytoplasmic staining" or a combination thereof. Specific staining typically refers to intense stain-colored loci in the microscopic image of the tissue, while as cytoplasmic staining refers to a continuous low intensity stain-color distributed in the interior of the cells. It is commonly believed that a "specific staining" is related to staining of the receptor and that "cytoplasmic staining" refers to unspecific staining or incomplete wash. Under the assumption that the specific staining and the cytoplasmic unspecific staining are inherently different it is probable that the interaction characteristics of the specific staining procedure and the cytoplasmic staining procedure. Therefore, the specific and cytoplasmic staining may be represented as two different features in a multidimensional fingerprint (e.g. two different peaks in an Interaction Map), when IHC is conducted in a time resolved manner. The same applies to other types of staining, including but not limited to "membrane staining" and "periplasmic staining". The ability to distinguish between different types of staining based on their interaction characteristics will reduce the need for manual ocular inspection of the specimens in microscope by a specialist in pathology and reduce cost and time in the evaluation. The process of separating the signals from different types of staining may further be automated so as to completely remove a subjective ocular assessment of a tissue sample.

The fact that an oncogene product can be presented in different manners and that the corresponding interaction maps are different have been discussed for measurements on living cell culture in the report "Gefitinib induces epidermal growth factor receptor dimers which alters the interaction characteristics with 125I-EGF." by Björkelund H, Gedda L, Barta P, Malmqvist M, Andersson K published in PLoS One, 2011;6(9):e24739, which is incorporated by reference herein. In this report, the interaction map for ¹²⁵I-EGF binding to the oncogene product EGFR in living A431 cells is greatly affected if the A431 cells are pretreated with the anticancer drug gefitinib. Gefitinib treatment results in one additional interaction (seen as one additional peak in the interaction map).

Disease related targets are sometimes related to aggregates. It is for example known that the aggregation of Amyloid beta peptides is frequent in Alzheimers disease, as evident in the report "Amyloid and Alzheimer's disease: inside and out." by Tam JH, Pasternak SH. as published in Can J Neurol Sci. 2012 May;39(3):286-98, which is incorporated by reference herein. Thus, the disease related targets need not to be different proteins, but can also include various forms of aggregates of proteins.

### EXAMPLE 1

In the following example, it is shown that the current invention can be applied to tissue samples from breast cancer patients and provide useful information.

Eight petri-dishes were prepared for an interaction measurement in LigandTracer Grey (Ridgeview Instruments AB, Uppsala, Sweden) of one probe with three different tissue samples placed in the same dish. Each petri dish contained one positive reference sample, one negative reference sample and one patient tissue sample, as illustrated in figure 3. Thus, in total eight tissue samples from different breast cancer patients were characterized. The patient material was extracted after obtaining a permit from the Regional Ethical Review Board in Uppsala, Sweden (DNr 2010/273).

The probe used in this example was a polyclonal antibody directed against the oncogene product HER2 (A0485, DAKO). To enable detection using LigandTracer Grey, the antibody was radiolabeled with 125-iodine using the standard Chloramine-T method. The positive reference tissue was a pellet of SKOV3 cells (known to express large quantities of HER2) and the negative reference sample was a liver from mouse. The experimental details of the measurement described in this example closely match the experimental details described in the report "Real-time immunohistochemistry analysis of embedded tissue" by Gedda L, Björkelund H, and Andersson K, published in Applied Radiation and Isotopes 68 (2010) 2372-2376, which is incorporated by reference herein.
The patient tissue samples were selected based on how the hospital originally classified the tissue samples in classical IHC staining of HER2 receptors. Two tissue samples were classified as negative, one tissue sample as ++/ FISH -, three as ++/ FISH + and two as +++.

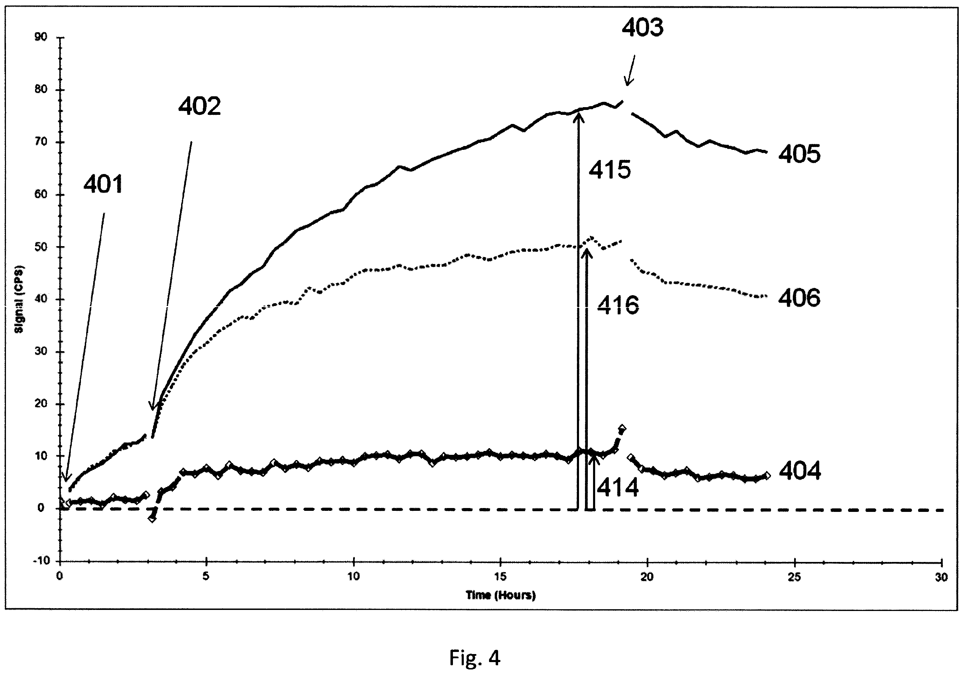

The eight petri dishes were run in LigandTracer Grey, first with an antibody concentration of 2 nM during 3 hours, followed by an antibody concentration of 6 nM during 16 hours, followed by dissociation in liquid devoid of antibody during 5 hours. An example of the output from the interaction analysis for one patient is shown in figure 4, wherein the time points for addition of 2 nM antibody (401), 6 nM antibody (402) and liquid exchange (403) are indicated by arrows. Three curves were produced during the measurement, one for the negative reference sample (404), one for the positive reference sample (405) and one for the patient tissue sample (406). The approximate signal levels for the three curves, indicated by arrows 414, 415, 416, was in this particular case 11 counts per seconds (CPS) for curve 404 (arrow 414), 76 CPS for curve 405 (arrow 415), and 50 CPS for curve 406 (arrow 416). The relative binding level of the patient sample compared to the positive reference sample was in this particular case 0.66 = 50/76. Measurements performed in LigandTracer Grey will have a signal magnitude which partly depends on the size of the tissue samples. All samples in this study had comparable size. Most importantly, the curvature of the results obtained in LigandTracer is independent of the size of the tissue sample.

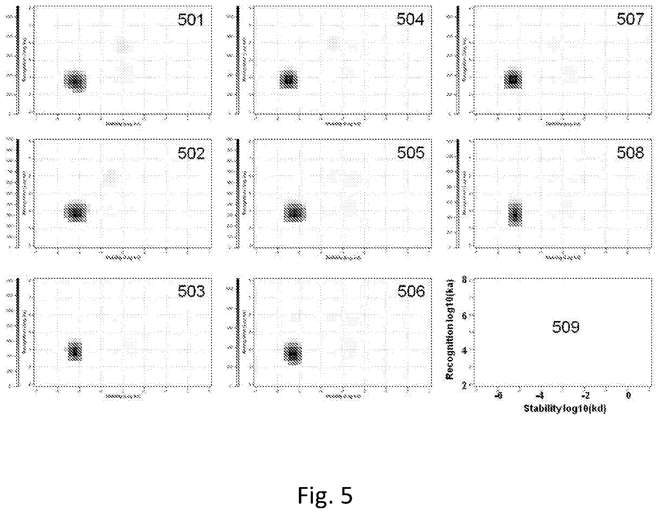

The acquired binding curves were subjected to interaction map analysis. It is important to emphasize that all interaction map calculations are conducted on binding curves normalized for signal magnitude, hence producing a map illustrating the relative proportions of the underlying processes. All interaction maps presented in this patent application have the same scale and axis labels, as illustrated in figure 5, item 509. All interaction maps shown in this patent application are normalized, i.e. if two measured binding curves differ only by a scaling factor, they will get equal interaction maps. Thus, the binding level per se is a suitable additional piece of information in such cases. It is however possible to use non-normalized interaction maps wherein the signal magnitude information is conserved, but such interaction maps are not simple to visualize in a clear manner. Figure 5 further shows that the interaction maps from the eight positive reference samples (501-508) were very similar. This is also seen in Table 1, wherein the peak positions for the eight positive reference samples are presented. All positive reference samples have one dominant peak at position log10(ka) = 3.8 ±0.11; log10(kd) = -5.25 ± 0.22, said peak contributing to 81-92% of the recorded signal.

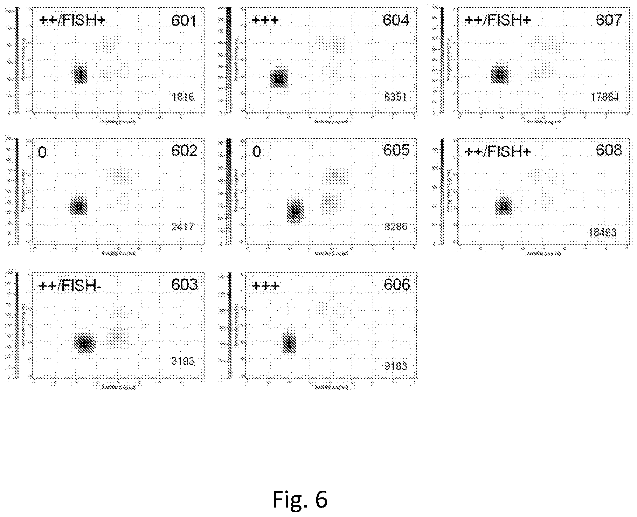

Figure 6 shows the interaction maps from the patient tissue samples, and in Table 1 the peak positions and peak contributions are presented. Interaction maps for tissue samples 604, 606, 607 are similar to the positive references both in terms of peak position, peak contribution. The tissue samples 602 and 608 produced Interaction Maps that are close to the positive reference peaks, and the remaining tissue samples 601, 603, 605 are more distant from the positive reference samples. Table 1 also presents the ratio of the patient tissue sample binding curve magnitude (i.e. maximum signal level) and the positive reference tissue sample binding curve magnitude as measured in the same dish (tissue samples 501 and 601 were measured simultaneously, 502 and 602 were measured simultaneously, etc). Keeping in mind that the interaction maps shown in Figure 5 and 6 are normalized to enhance visibility, patient sample 607 had a signal level corresponding to 64% of the positive reference measured in the very same dish. This means that not only did patient sample 607 correspond to the type of interaction as estimated using the position of the peak in the interaction map, patient sample 607 did also express a large quantity of HER2. For patient sample 604, the interaction map peak position was similar to the average positive reference sample, but it did not produce as high signal (only 24% of the positive reference sample in the same dish). For patient sample 603, the interaction map produced a different peak position, but a clear signal level (32%), suggesting that the properties of the HER2 expression in patient sample 603 is different than in the positive reference tissues, but similar enough to allow the probe antibody to interact with the receptor. The interaction map for 603 further has two peaks, one dominant and one weak, which again indicates that the expression of HER2 on the tissue sample from patient 603 is different from the positive reference sample.

In this particular example, it would be advisable to extract a limited region of the interaction map for classification. The peak that is corresponding to the major binding event as defined by the positive reference tissue is located at position log10(ka) = 3.8 ±0.11; log10(kd) = -5.25 ± 0.22. By including all peaks that are approximately one unit in distance from the reference tissue peak, all relevant variations for the interaction of the antiHER2 antibody with HER2 would likely be captured. The suggested region to extract data from in this case is hence log10(ka) between 3 and 5 and log10(kd) between -6 and -4. This region is denoted log10(ka) : [3 , 5] and log10(kd) : [-6 , -4]. Another possible region for this particular example is log10(ka) : [3.3 , 4.3] and log10(kd) : [-5.75 , -4.75].

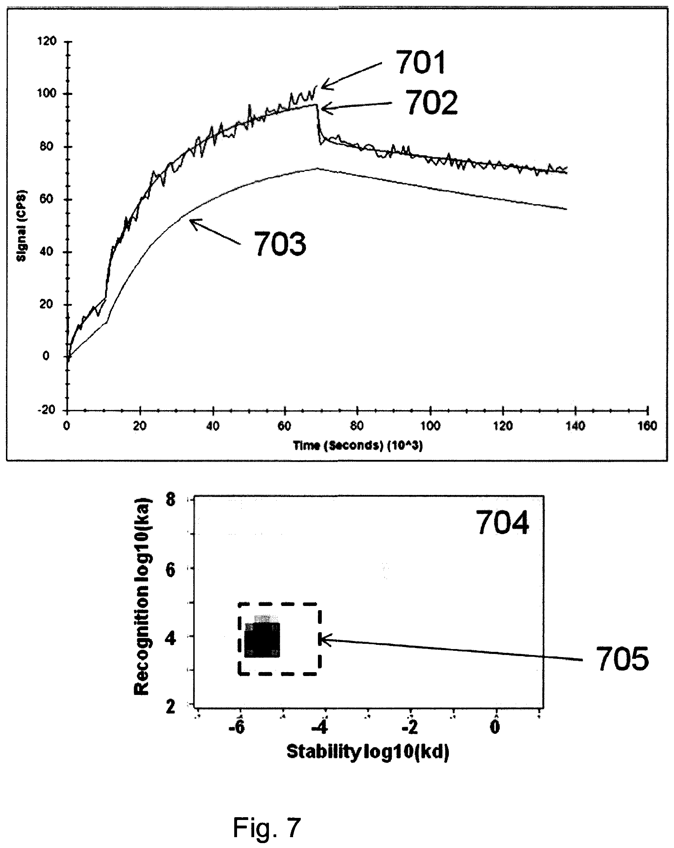

It is possible to use the region log10(ka) : [3 , 5] and log10(kd) : [-6 , -4] of an interaction map to calculate the estimate binding curve corresponding to the interactions in said region. As illustrated in Figure 7, the measured binding curve 701 (noisy curve) is approximated with a sum of primitive curves into an interaction map (704), and the curve corresponding to the complete interaction map (702) closely matches the measured curve (701). If a region is defined in the interaction map (705), said region containing an interaction of particular importance, it is possible to create the estimated binding curve originating from said region (by calculating the sum of the primitive curves in said region only). The binding curve (703) corresponding to interaction within region 705 is the dominant contributor in this particular case, but approximately 25% of the signal originates from interactions described in other parts of the interaction map. Hence, by defining a region it is possible to exclude the impact of potentially disturbing parallel interactions of the probe with structures in the tissue sample.

In this particular example, the column Weight in Table 1 is an estimation of the contribution of the major peak, which in turn is a good estimation of the contribution of the interactions from the region log10(ka) : [3 , 5] and log10(kd) : [-6 , -4]. As seen in Table 1, as high as 92% and as low as 58% of the detected signal may originate from this region.

**Table 1**

| PID | Type | Relative Signal Value | Pos. log ka | Pos. log kd | Weight (%) |
|---|---|---|---|---|---|
| 501 | PosRef | 1 | 3.74 | -5.17 | 81 |
| 502 | PosRef | 1 | 3.87 | -5.15 | 87 |
| 503 | PosRef | 1 | 3.87 | -5.21 | 90 |
| 504 | PosRef | 1 | 3.83 | -5.50 | 87 |
| 505 | PosRef | 1 | 3.85 | -5.22 | 84 |
| 506 | PosRef | 1 | 3.75 | -5.30 | 88 |
| 507 | PosRef | 1 | 3.83 | -5.30 | 87 |
| 508 | PosRef | 1 | 3.76 | -5.20 | 92 |
| 601 | ++/F+ | 0.41 | 4.11 | -4.81 | 77 |
| 602 | 0 | 0.36 | 4.07 | -4.90 | 76 |
| 603 | ++/F- | 0.32 | 3.91 | -4.62 | 67 |
| 604 | +++ | 0.24 | 3.86 | -5.49 | 76 |
| 605 | 0 | 0.07 | 3.79 | -4.72 | 58 |
| 606 | +++ | 0.44 | 3.91 | -5.00 | 86 |
| 607 | ++/F+ | 0.64 | 4.07 | -5.10 | 77 |
| 608 | ++/F+ | 0.10 | 4.06 | -4.90 | 85 |

This example shows that interaction map can repeatedly produce similar values for the positive control. It also shows that the patient tissue samples have different properties compared to the positive control, except for a few that were previously classified as +++ or ++/FISH+ in traditional analysis. It additionally shows that the signal intensity for the patient tissue samples as related to the positive control in the same dish vary significantly and in a manner where a high signal intensity can be related to a negative sample (602 and 603 in Table 1) and vice versa (604 and 608 in Table 1), meaning that the signal intensity alone is insufficient for discriminating the patient tissue expressing high amounts of HER2 from the ones low amounts of HER2.

It would also be possible to use the dominant peak position of the positive control as the definition of where to identify the peak position in the tissue sample mounted onto the same solid support. With such a strategy, one would first determine if the positive control measurement is within predefined limits, for example regarding signal intensity and binding curve continuity, and in case the positive control measurement does not meet the predefined limits the complete measurement is rejected due to insufficient data quality. If the data quality is acceptable), one would then identify the peak position in the interaction map of the positive control and use said position as a reference position, then identify the peak in the interaction map for the tissue sample which is closest to the reference position and which has a weight greater than 10%, and finally extract the weight for the identified peak to be used as characteristic value. When applying such a procedure, a high characteristic value (greater than approximately 75%) would indicate that the tissue sample is expressing large quantities of the HER2 receptor, information which in turn can be used to select a suitable therapy for the human from which the tissue sample was originally taken.

### EXAMPLE 2

In the following example, it is shown that the current invention can be applied to tissue samples from breast cancer patients and provide information related to the expression level of the HER2 receptor in said patient sample.

The data set used in example 1 was later supplemented with another 12 patient samples, leading to a total of 20 different patient samples being included in the data. The patient samples were selected based on the original assessment on HER2 expression as made by the routine laboratory at the hospital from which said samples originated. Of these 20 patient samples, four were known not to express HER2 in any significant manner (score 0 in Herceptest IHC procedure), four were known to express small quantities of HER2 (score + in Herceptest IHC procedure), four were known to express moderate quantities of HER2, but later tested negative using fluorescence in-situ hybridization (FISH) analysis (score ++ in Herceptest IHC procedure, - in FISH), four were known to express moderate quantities of HER2, and were later confirmed positive using FISH analysis (score ++ in Herceptest IHC procedure, + in FISH), and four were known to express large quantities of HER2 (score +++ in Herceptest IHC procedure, - in FISH). The binding of polyclonal antibody directed against the oncogene produce HER2 (A0485, DAKO), labeled with 125-I was quantified as described in Example 1. All binding curves were subjected to Interaction Map analysis as described in Example 1. For analysis, the Weight%, i.e. the relative contribution of the weights of one defined peak in relation to all weights, was calculated for the dominant peak and for the second-most contributing peak. The resulting weight assessments are shown in Table 2.

**Table 2**

| ID | Category | Peak1 W% | Peak2 W% |
|---|---|---|---|
| Pat01+++ | A | 87.44 | 3 |
| Pat02+++ | A | 85.59 | 4.74 |
| Pat03+++ | A | 82.19 | 4.8 |
| Pat04+++ | A | 85.47 | 3.96 |
| Pat05++/+ | A | 76.69 | 7.11 |
| Pat06++/+ | A | 84.65 | 3.78 |
| Pat07++/+ | A | 72.42 | 14.87 |
| Pat08++/+ | A | 77.25 | 6.99 |
| | | | |
| Pat09++/- | B | 46.29 | 23.65 |
| Pat10++/- | B | 61.93 | 13.51 |
| Pat11++/- | B | 67.47 | 17.06 |
| Pat12++/- | B | 24.82 | 66.15 |
| Pat13+ | B | 52.59 | 28.04 |
| Pat14+ | B | 45.54 | 38.98 |
| Pat15+ | B | 45.05 | 35.42 |
| Pat16+ | B | 70.67 | 18.94 |
| Pat17_0 | B | 62.17 | 28.14 |
| Pat18_0 | B | 58.08 | 17.12 |
| Pat19_0 | B | 33.47 | 52.33 |
| Pat20_0 | B | 76.14 | 12.3 |
| | | | |
| Average Category A | | 81.46 | 6.16 |
| Standard deviation Category A | | 5.37 | 3.81 |
| Average Category B | | 53.70 | 29.30 |
| Standard deviation Category B | | 15.32 | 16.51 |
| | | | |
| P-value (A is equal to B) | | 0.000040 | 0.00046 |

The Weight% for the dominant, most contributing peak in the Interaction Map (denoted "Peak 1 W%") proved to be different depending on the HER2 expression status of the patient sample, as seen in Table 2. For patient samples scoring +++ or ++/+ (collectively denoted category A, also known as the case group), i.e. patient sample known in advance having large amounts of HER2 expressed, the Peak1 W% averaged at 81.46% (with a standard deviation of 5.37). For patient samples scoring 0, +, or ++/- (collectively denoted category B, also known as the control group), i.e. patient samples known in advance having no or small amounts of HER2 expressed, the Peak1 W% averaged at 53.70% (with a standard deviation of 15.32). The probability that category A and category B share the same statistical distribution is P=0.000040 (using a two-tailed T-test). If the same type of analysis is conducted on the weight% of the second-most contributing peak (denoted Peak2 W% in table 2), category A has an average of 6.16% and category B an average of 29.30%, which also is significantly different (P=0.00046) as estimated using a two-tailed T-test. In plain words, this means that for patient samples of category A, the Interaction Map contains one dominant peak and almost nothing else, while as for patient samples of category B, there are at least two different peaks in the Interaction Map. This can be seen in Figure 6, wherein for example patient samples 601 and 608 (belonging to category A) have a single dominant peak, while as patient samples 603 and 605 (belonging to category B) have clear secondary peaks.

According to current clinical practice, the case group in this example correspond to individuals to which the therapeutic antibody Herceptin® would be prescribed. The control group correspond to individuals to which the therapeutic antibody Herceptin® would NOT be prescribed. Hence, the primary peak was related to HER2 expression as determined by IHC and the secondary peaks were related to other events, potentially cytoplasmic staining or other irrelevant interactions. Importantly this relates to relative abundances of the primary HER2 peak and the additional binding events identified in the binding curve.

This example thus shows that the Interaction Map procedure can produce results which in turn can be used to estimate if a patient sample has high expression levels of the HER2 oncogene product.

### EXAMPLE 3

In the following example, it is shown that the current invention can be applied to tissue samples using antibody probes from a different vendor.

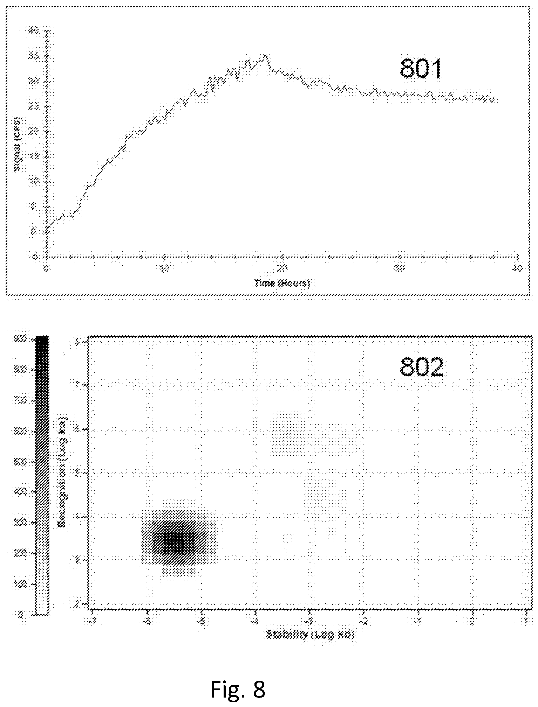

Two petri-dishes were prepared for an interaction measurement in LigandTracer Grey (Ridgeview Instruments AB, Uppsala, Sweden) essentially as described in Example 1. The probe used in this example was a polyclonal antibody directed against the oncogene produce HER2 (Atlas Antibodies, product number HPA001383, Lot Number A69483). To enable detection using LigandTracer Grey, the antibody was radiolabeled with 125-iodine using the standard Chloramine-T method. As shown in Figure 8, the resulting binding trace (801) indicates that the antibody HPA001383 binds to HER2 in the positive control tissue, and said binding trace can be evaluated using the Interaction Map tool (802).

This example thus shows that the invention in not limited to any particular antibody supplier.

Although the invention has been described with regard to its preferred embodiment, which constitute the best mode currently known to the inventor, it should be understood that the invention is defined according to the appended claims.

## Claims

1. A method for detecting a disease in a mammal, comprising:
providing a probe specific for a disease-related target of a tissue sample;
performing a time resolved measurement of the interaction between said probe and said disease-related target;
computing in a processor a multidimensional normalized fingerprint representation of said time resolved measurement;
**characterized by**
extracting data from a predefined region of said multidimensional fingerprint;
wherein the pre-defined region is defined based on one or more analyses of a positive control known to express said disease-related target;
processing the data from said pre-defined region of said multidimensional fingerprint in a processor by applying a classification algorithm which uses information within said region of said fingerprint to determine the presence and/or the quantity of said disease-related target and the association rate or dissociation rate of said probe and disease-related target; and
wherein said multidimensional fingerprint is obtained using the so-called "Interaction Map" method to obtain a representation of said time resolved measurement of the interaction of said probe with said disease-related target, wherein said representation is displayed as a topographic map, wherein each peak corresponds to association and dissociation rate values, which means that said time resolved measurement is composed of an interaction of said probe (121) with said disease-related target of said tissue sample (122); and
wherein said time resolved measurement is conducted in one of
(a) a flow cytometer; or
(b) an instrument comprising a solid support onto which the biological samples are attached, and wherein at least one positive control and one biological sample are attached to said solid support, and wherein a defined feature in the multidimensional fingerprint of said positive control is used to determine the defined region for which the multidimensional fingerprint is evaluated for the biological sample;
and wherein said time resolved measurement comprises:
attaching said biological tissue sample to a solid support;
providing a solution of a probes of interest;
bringing said solution in contact with said biological tissue sample attached to said support;
detecting the presence of interaction between probe and biological tissue sample and the rate of formation of probe and disease related target complexes to form a curve representing the amount of probe bound to the disease related target over time;
and wherein two or more biological samples are attached to non-overlapping areas on said solid support; at least one biological sample is a positive reference sample; and the detection of the interaction between probe and biological samples are conducted simultaneously; and
wherein said probe is labelled with a detectable marker.

2. The method according to claim 1, wherein the relative abundances of the receptor plethora of a the cells comprising said biological tissue sample is represented in said multidimensional fingerprint.

3. The method as claimed in any preceding claim, wherein said probe is an antibody.

4. The method as claimed in claim 1, said method further comprising: said probe being an antibody probe known to interact with HER2, said antibody probe possibly being labeled with a detectable marker; determining in said processor the weight and the position of the three most contributing peaks in said Interaction Map; performing in the processor an estimation of the level of expression of HER2 by comparing the weight for the dominant peak to the weight of the other peaks.

5. The method as claimed in any of the previous claims, wherein said region of the interaction map is log10(ka) : [3 , 5] and log10(kd) : [-6 , -4] (Fig. 8).

6. The method according to any preceding claim, wherein said disorder is selected from a neurological disorder, a cancer or diabetes.

## Patentansprüche

1. Verfahren zum Erfassen einer Erkrankung bei einem Säugetier, umfassend:
Bereitstellen einer für ein erkrankungsbezogenes Target einer Gewebeprobe spezifischen Sonde;
Durchführen einer zeitaufgelösten Messung der Wechselwirkung zwischen der Sonde und dem erkrankungsbezogenen Target;
Errechnen einer mehrdimensionalen normalisierten Fingerabdruckdarstellung der zeitaufgelösten Messung in einem Prozessor;
**gekennzeichnet durch**
Extrahieren von Daten aus einer vorbestimmten Region des mehrdimensionalen Fingerabdrucks;
wobei die vorbestimmte Region auf einer oder mehreren Analysen einer positiven Kontrolle beruht, von der bekannt ist, dass sie das erkrankungsbezogene Target exprimiert;
Verarbeiten der Daten aus der vorbestimmten Region des mehrdimensionalen Fingerabdrucks in einem Prozessor durch Anwenden eines Klassifizierungsalgorithmus, der Informationen innerhalb der Region des Fingerabdrucks dazu verwendet, das Vorhandensein und/oder die Menge des erkrankungsbezogenen Targets und die Assoziationsrate oder Dissoziationsrate der Sonde und des erkrankungsbezogenen Targets zu bestimmen; und
wobei der mehrdimensionale Fingerabdruck unter Verwendung der sogenannten Methode der "Wechselwirkungskarte" erhalten wird, um eine Darstellung der zeitaufgelösten Messung der Wechselwirkung der Sonde mit dem erkrankungsbezogenen Target zu erhalten, wobei die Darstellung als topografische Karte angezeigt wird, wobei jeder Peak Assoziations- und Dissoziationsratenwerten entspricht, was bedeutet, dass die zeitaufgelöste Messung sich aus einer Wechselwirkung der Sonde (121) mit dem erkrankungsbezogenen Target der Gewebeprobe (122) zusammensetzt; und
wobei die zeitaufgelöste Messung in einem der folgenden ausgeführt wird:
(a). einem Durchflusszytometer; oder
(b). einem Instrument, das einen festen Träger umfasst, an dem die biologischen Proben befestigt sind, und wobei zumindest eine positive Kontrolle und eine biologische Probe an dem festen Träger befestigt sind, und wobei ein definiertes Merkmal in dem mehrdimensionalen Fingerabdruck der positiven Kontrolle dazu verwendet wird, die definierte Region, für die der mehrdimensionale Fingerabdruck bewertet wird, für die biologische Probe zu bestimmen;
und wobei die zeitaufgelöste Messung Folgendes umfasst:
Befestigen der biologischen Gewebeprobe an einem festen Träger;
Bereitstellen einer Lösung einer Sonde von Interesse;
Herstellen eines Kontakts zwischen der Lösung und der an dem festen Träger befestigten biologischen Gewebeprobe;
Erfassen des Vorhandenseins einer Wechselwirkung zwischen Sonde und biologischer Gewebeprobe und der Rate der Bildung von Komplexen zwischen Sonde und erkrankungsbezogenem Target, um eine Kurve zu bilden, die die Menge an Sonde, die an das erkrankungsbezogene Target gebunden ist, im Zeitverlauf darstellt;
und wobei zwei oder mehr biologische Proben an einander nicht überlappenden Bereichen des festen Trägers befestigt sind; zumindest eine biologische Probe eine positive Referenzprobe ist; und die Erfassung der Wechselwirkung zwischen Sonde und biologischen Proben gleichzeitig ausgeführt werden; und
wobei die Sonde mit einem nachweisbaren Marker markiert ist.

2. Verfahren nach Anspruch 1, wobei das relative Vorkommen der Rezeptorfülle der Zellen, die die biologische Gewebeprobe umfassen, in dem mehrdimensionalen Fingerabdruck dargestellt ist.

3. Verfahren nach einem der vorstehenden Ansprüche, wobei die Sonde ein Antikörper ist.

4. Verfahren nach Anspruch 1, wobei das Verfahren weiter Folgendes umfasst: dass die Sonde eine Antikörpersonde ist, von der bekannt ist, dass sie mit HER2 wechselwirkt, wobei die Antikörpersonde möglicherweise mit einem nachweisbaren Marker markiert ist; Bestimmen des Gewichts und der Position der drei am meisten beitragenden Peaks in der Wechselwirkungskarte in dem Prozessor; Durchführen einer Schätzung des Ausmaßes an Expression von HER2 in dem Prozessor durch Vergleichen des Gewichts beim dominanten Peak mit dem Gewicht der anderen Peaks.

5. Verfahren nach einem der vorstehenden Ansprüche, wobei die Region der Wechselwirkungskarte log10(ka) : [3,5] und log10(kd) : [-6,-4] (Fig. 8) ist.

6. Verfahren nach einem der vorstehenden Ansprüche, wobei die Störung aus einer neurologischen Störung, einem Krebs oder Diabetes ausgewählt ist.

## Revendications

1. Procédé de détection d'une maladie dans un mammifère, comprenant :
fournir une sonde spécifique d'une cible liée à la maladie d'un échantillon de tissu ;
effectuer une mesure résolue en temps de l'interaction entre ladite sonde et ladite cible liée à la maladie ;
calculer dans un processeur une représentation d'empreinte digitale normalisée multidimensionnelle de ladite mesure résolue en temps ;
**caractérisé par**
extraire des données issues d'une région prédéfinie de ladite empreinte digitale multidimensionnelle ;
dans lequel la région prédéfinie est définie sur la base d'une ou plusieurs analyses d'un contrôle positif connu pour exprimer ladite cible liée à la maladie ;
traiter les données issues de ladite région prédéfinie de ladite empreinte digitale multidimensionnelle dans un processeur en appliquant un algorithme de classification qui utilise des informations au sein de ladite région de ladite empreinte digitale pour déterminer la présence et/ou la quantité de ladite cible liée à la maladie et le taux d'association ou taux de dissociation desdites sonde et cible liée à la maladie ; et
dans lequel ladite empreinte digitale multidimensionnelle est obtenue en utilisant le procédé dit « Carte d'Interaction » (« Interaction Map ») pour obtenir une représentation de ladite mesure résolue en temps de l'interaction de ladite sonde avec ladite cible liée à la maladie, dans lequel ladite représentation est affichée sous forme d'une carte topographique, dans laquelle chaque pic correspond à des valeurs de taux d'association et de dissociation, ce qui signifie que ladite mesure résolue en temps est composée d'une interaction de ladite sonde (121) avec ladite cible liée à la maladie dudit échantillon de tissu (122) ; et
dans lequel ladite mesure résolue en temps est effectuée dans l'un de
(a) un cytomètre de flux ; ou
(b) un instrument comprenant un support solide sur lequel sont attachés les échantillons biologiques, et dans lequel au moins un contrôle positif et un échantillon biologique sont attachés audit support solide, et dans lequel une caractéristique définie dans l'empreinte digitale multidimensionnelle dudit contrôle positif est utilisée pour déterminer la région définie pour laquelle l'empreinte digitale multidimensionnelle est évaluée pour l'échantillon biologique ;
et dans lequel ladite mesure résolue en temps comprend :
attacher ledit échantillon de tissu biologique à un support solide ;
fournir une solution d'une sonde d'intérêt ;
mettre en contact ladite solution avec ledit échantillon de tissu biologique attaché audit support ;
détecter la présence d'une interaction entre sonde et échantillon de tissu biologique et le taux de formation de complexes de sonde et de cible liée à la maladie pour former une courbe représentant la quantité de sonde liée à la cible liée à la maladie dans le temps ;
et dans lequel deux ou plusieurs échantillons biologiques sont attachés à des zones non chevauchantes sur ledit support solide ; au moins un échantillon biologique est un échantillon de référence positif; et la détection de l'interaction entre sonde et échantillons biologiques sont menées
simultanément ; et
dans lequel ladite sonde est repérée avec un marqueur détectable.

2. Procédé selon la revendication 1, dans lequel les abondances relatives de la pléthore du récepteur des cellules comprenant ledit échantillon de tissu biologique est représentée dans ladite empreinte digitale multidimensionnelle.

3. Procédé tel que revendiqué dans l'une quelconque des revendications précédentes, dans lequel ladite sonde est un anticorps.

4. Procédé tel que revendiqué dans la revendication 1, ledit procédé comprenant en outre : ladite sonde étant une sonde anticorps connue pour interagir avec le HER2, ladite sonde anticorps étant possiblement repérée avec un marqueur détectable ; déterminer dans ledit processeur le poids et la position des trois pics les plus contributifs dans ladite Carte d'Interaction ; effectuer dans le processeur une estimation du niveau d'expression du HER2 en comparant le poids pour le pic dominant au poids des autres pics.

5. Procédé tel que revendiqué dans l'une quelconque des revendications précédentes, dans lequel ladite région de la carte d'interaction est log10(ka) : [3 , 5] et log10(kd) : [-6 , -4] (Fig.8).

6. Procédé selon l'une quelconque des revendications précédentes, dans lequel ledit trouble est sélectionné parmi un trouble neurologique, un cancer ou le diabète.
